# EUROPEAN PATENT SPECIFICATION

(11) **EP 1 643 837 B1**
(45) Date of publication and mention of the grant of the patent: **06.05.2009**
(21) Application number: 04778209.9
(22) Date of filing: 12.07.2004
(51) Int. Cl.: A01N 43/84, A61L 2/00

(54) **DOUBLE DYE METHOD TO INACTIVATE BLOOD BORNE PATHOGENS**
DOPPELTES FÄRBEVERFAHREN ZUR INAKTIVIERUNG VON HÄMATOGENEN KRANKHEITSERREGERN
PROCEDE A DEUX TEINTURES POUR INACTIVER DES PATHOGENES VEHICULES PAR LE SANG

(30) Priority: 11.07.2003 US 487160 P
(43) Date of publication of application: 12.04.2006
(73) Proprietor: Shanbrom Technologies, LLC, Ojai, CA 93023-3732 (US)
(72) Inventor: SHANBROM, Edward, Santa Ana, CA 92705 (US)
(74) Representative: Fiener, Josef
(86) International application number: PCT/US2004/022583
(87) International publication number: WO 2005/006861

(56) References cited:
- WO-A-00/03751
- WO-A-95/32732
- WO-A-99/13921
- DOCAMPO R ET AL: "ENHANCEMENT OF THE CYTOTOXICITY OF CRYSTAL VIOLET AGAINST TRYPANOSOMA-CRUZI IN THE BLOOD BY ASCORBATE" MOLECULAR AND BIOCHEMICAL PARASITOLOGY, vol. 27, no. 2-3, 1988, pages 241-247, XP002309426 ISSN: 0166-6851
- PATENT ABSTRACTS OF JAPAN vol. 006, no. 114 (C-110), 25 June 1982 (1982-06-25) & JP 57 042625 A (IDOTA YUTAKA), 10 March 1982 (1982-03-10) -& DATABASE WPI Section Ch, Week 198216 Derwent Publications Ltd., London, GB; Class B05, AN 1982-31666E XP002309427 & JP 57 042625 A (IDOTA H) 10 March 1982 (1982-03-10)

## Description

### Background of the Invention

### Area of the Art

The present invention deals with disinfecting blood and blood fractions and more specifically with a simple method to destroy a large number of pathogens in human blood prior to transfusion.

### Description of the Prior Art

A wide variety of treatments have been used to reduce or eliminate infectious agents from blood and blood products. Although the so-called disinfecting dyes have been known for a long time, there use has not been widespread except for parasite reduction in certain tropical countries. It has been known that certain dyes combined with actinic light treatments can result in inactivation of a number of different virus. However, the requirement for light exposure has limited the acceptance of such methods both because light treatments are complex and because such treatments may result in significant collateral damage.

WO 00/03751 describes a method for disinfecting a blood sample using visible light instead of ultraviolet light in which the dyes with "disinfectant" properties, e.g. a mixture of methylene blue and crystal violet, are afterwards removed by filtering the sample through a polyvinyl acetal filter.

From WO 95/32732 it is known that ascorbate added sequentially or simultaneously with methylene blue to blood plasma has a beneficial and stabilising effect on the treated blood plasma.

R. Docampo et al. (Molecular and Biochemical Parasitology, 27 (1988) 241-247) teach that ascorbate enhances the activity of crystal violet treatment of blood against *Trypanosoma cruzi* the causative agent of Chagas' disease.

### Summary of the Invention

Treatment of blood and blood fractions with a mixture of methylene blue and crystal violet ("Double-Dye") has been found to eliminate a variety of bacteria, virus and parasites without appreciably damaging blood cells or proteins. A 4-log reduction in *Staphylococcus epidermidis, Staphylococcus aureus, Bacillus subtilis, Escherichia coli, Yersinia enterocolitica, Pseudomonas aeruginosa, Pseudomonas fluorescensand Serratia marcescens* was observed. The "Double-Dye" method yielded a 9-log reduction of vesicular stomatitis virus, 6-log reduction of encephalomyocarditis virus, 4.5-log reduction of bovine diarrheal virus and a 5-log reduction of porcine parvovirus. Complete kills of these viruses were achieved by adding sufficient ascorbate to largely convert the two dyes into their leuco forms. While other anti-oxidants are effective, ascorbic acid appears the most efficacious at inducing electron transfer, reducing the dyes to a leuco form. Finally, a PVA filtration method is used to remove the dyes before use of the disinfected blood or blood component. Thus, the Double-Dye-ascorbate-PVA method is an effective, low-cost way to eliminate pathogens from whole blood, platelets, plasma or other blood fractions without incurring risks inherent in using ultraviolet light.

### Detailed Description of the Invention

The following description is provided to enable any person skilled in the art to make and use the invention and sets forth the best modes contemplated by the inventor of carrying out his invention. Various modifications, however, will remain readily apparent to those skilled in the art, since the general principles of the present invention have been defined herein specifically to provide an enhanced dye-based method for disinfecting blood and blood products.

### Dye Treatment

The solution to be treated is brought to about 0.01% by weight double dye (methylene blue plus crystal violet) by adding a stock solution containing equal weight percentages of the two dyes. For example, a double dye stock solution of 1% by weight of each of the dye may be conveniently used. Further a reducing agent is added. The best results are obtained by adding sufficient reducing agent in the form of ascorbic acid (vitamin C) or its close derivatives to result in the diluted dye solution being decolorized. Generally, a weight percentage of added ascorbate equal to the combined weights of the added dye is sufficient. In some cases, the blood sample may be especially poor in natural reducing agents (as indicated by an obvious blue color after dye and ascorbate addition) so that additional ascorbate must be added to reduce the color.

### Dye Removal

Although the dyes are considered safe, it is considered prudent to remove them before administering the treated blood or blood fraction to a patient. This can be accomplished by a variety of filters and binding materials. In the present invention removal with polyvinyl acetal is a preferred method although the other removal methods well known to the art are also useable. For test purposes, 2.5ml of 1% "Double-Dye" solution (crystal violet/methylene blue, equal weights) was added to 250ml whole blood (final dye concentration 0.01% by weight of each dye). For this experiment the dye was not decolorized with ascorbate. A sample of the blood was centrifuged to pellet the blood cells, and the absorbance ("optical density" or O.D.) of the supernatant plasma was determined at 600nm and 660nm.

The blood was then filtered through a polyvinyl acetal (PVA) sponge filter column (six 1.0cm thick pads) in order to remove the dyes-both of which bind strongly to the PVA material. A second aliquot of the blood was centrifuged, and the absorbance of the filtered supernatant plasma was measured at 600nm and 660nm as a blanking control. The results show essentially total removal of the dyes.

The absorbance results are listed below in Table 1:

**Table 1**

| Pre | O.D. |
|---|---|
| 600nm | 0.65 |
| 660nm | 0.82 |
| | |

| Post | |
|---|---|
| 600nm | 0.01 |
| 660nm | 0.01 |

### Double Dye Effect on Red Cells

It is imperative that any treatment aimed at disinfecting blood does not result in damage to delicate blood cells. In a typical experiment, 2.5ml of 1% "Doublo-Dye" solution (crystal violet/methylene blue) was added to 250ml whole blood (final dye concentration 0.01%). Sufficient reducing agent (ascorbate) was added to decolorize the dye (approximately 0.015% ascorbate by weight). An aliquot of 10.0ml of untreated blood was set aside as the normal control.

The blood was filtered through a PVA filter column (six 1.0cm thick pads), gravity flow. 10.0ml samples of control and treated blood were examined visually for presence of hemoglobin in the supernatant plasma and changes in packed cell volume (both signs of cell damage). A blood smear was prepared and the cells were examined microscopically for morphological changes.

Annexin-V binding capacity (%) of the RBCs was determined by FACS analysis as a measure of membrane alteration. Annexin-V binds to phosphatidyl serine, which is found predominantly on the inner surface of the red cell membrane. Thus, increased binding is a sign of membrane damage as the inner surface should not be accessible. The FACS and other results are shown below.

| Hemoglobin | |
|---|---|
| Control | neg |
| Treated | neg |
| | |

| Packed cell volume | |
|---|---|
| Control | 4.9ml |
| Treated | 4.9ml |
| | |

| Microscopic Appearance | |
|---|---|
| Control | normal |
| Treated | normal |

| Annexin-V Binding (%) (By FACS) | |
|---|---|
| Control | 6.25% |
| Treated | 6.31% |

### Effects on Platelets

1.0ml of 1% "Double-Dye" solution (crystal violet/methylene blue) was added to 100ml of platelet rich plasma (PRP) (final dye concentration 0.01%). 100ml of untreated PRP was set aside as the normal control. The PRP samples were incubated for nine days at 21°C with constant mixing.

On days 0, 3, 6 and 9, 50ml of the treated PRP was filtered through a PVA filter column (two 1.0cm thick pads), gravity flow. 0.1ml of the treated PRP was mixed with 1.0ml of fibrinogen, 0.1ml prothrombin complex and 0.01ml 0.025M CaCl2. This was repeated with the control PRP. Clot formation indicates normal platelet thromboplastin activity.

| Clot formation time (day 0) | |
|---|---|
| Control | 62 sec |
| Treated | 63 sec |

| Clot formation time (day 3) | |
|---|---|
| Control | 60 sec |
| Treated | 63 sec |

| Clot formation time (day 6) | |
|---|---|
| Control | 94 sec |
| Treated | 99 sec |

| Clot formation time (day 9) | |
|---|---|
| Control | 134 sec |
| Treated | 142 sec |

These results indicate that the treatment does not significantly damage the platelets as compared to untreated cells. Thus, the "Double-Dye" method is a safe, easy to perform method of inactivating pathogens in RBC and platelet suspensions.

### Parasites

Chagas disease is endemic to south and Central America and is caused by the parasite *Trypanosoma cr*uzi. About 100 million people live in conditions that put them at risk of the disease. Traditional treatment is with methylene blue and ultraviolet light. The ultraviolet light is absorbed by the dye molecules and the imparted energy damages/kills the parasite; however, this same energy elevates the risks of damage to blood proteins and may even pose a risk of carcinogenesis. Inadequate treatments for Chagas disease underscore the need for a blood supply free of pathogen contamination and affordable for developing countries. The current invention includes the discovery that the combining of methylene blue and crystal violet dyes allows *T. cruzi* to be destroyed in whole blood or plasma without the need of ultraviolet light. Five ml of a 1.0 percent solution of methylene blue/crystal violet dyes (1% by weight of each dye) was added to 500 ml of whole blood (final dye concentration of 0.01%) and incubated in the presence of ascorbic acid (vitamin C) sufficient largely to decolorize the dye solution (as judged in the absence of blood). Blood was then passed through a polyvinyl acetal (PVA) sponge filter, which removed all visible dye. There was no injury to erythrocytes, as measured by morphologic examination and FACS (fluorescence activated cell sorting) measurement of annexin-V binding. Platelets also were "double-dye" treated and passed through the PVA filter. No loss of platelet function was observed. No evidence of *T. cruzi* was found in samples treated with the methylene blue/crystal violet dyes.

### Trypanosomal Inactivation

Approximately 500ml of fresh whole blood (CPD-A1 anticoagulant) was obtained. The blood was spiked with 1.0ml of *Trypanosoma cruzi* suspension (1x108 org./ml). Final concentration of *T. c*ruzi in the blood was 2x105 org/ml.

The spiked blood was mixed for 15 min. at 21°C. 250ml of the spiked blood was dispensed into another fresh blood bag containing 2.5ml (50mg) of the "Double Dye" (crystal violet/methylene blue) solution. Another 250ml of blood was untreated, and was used as the positive *Trypanosoma cruzi* control. Both bags were incubated at 5°C for 48hrs. At 48hrs, both samples were filtered through a column of six 1.0cm thick PVA filters to remove the dye mixture.

The samples were serially diluted and inoculated into BHI broth, and incubated for 28days. The cultures were examined microscopically on days 7, 14, 21 and 28 for evidence of live parasites as shown in Table 2.

**Table 2**

| No dye | Day 7 | Day 14 | Day 21 | Day 28 |
|---|---|---|---|---|
| Inoculum (org/ml) | | | | |
| 1x10¹ | neg | neg | neg | neg |
| 1x10² | neg | neg | neg | pos |
| 1x10³ | neg | neg | pos | pos |
| 1x10⁴ | neg | pos | pos | pos |
| 1x10⁵ | pos | pos | pos | pos |

| 0.2mg/g CV/MB | Day 7 | Day 14 | Day 21 | Day 28 |
|---|---|---|---|---|
| Inoculum (org/ml) | | | | |
| 1x10¹ | neg | neg | neg | neg |
| 1x10² | neg | neg | neg | neg |
| 1x10³ | neg | neg | neg | neg |
| 1x10⁴ | neg | neg | neg | neg |
| 1x10⁵ | neg | neg | neg | neg |

These results demonstrate that the double dye is completely effective in eliminating the parasite. Not shown in these results is the effect of ascorbate. Essentially, ascorbate had little effect on the destruction of the parasite. However, in actual practice one would desire the elimination of virus (see below) as well as parasites so that ascorbate addition would be preferred.

### Bacteriocidal Effect

Sixteen 10.0ml samples of whole blood were spiked with the following bacteria (final conc. 1x10⁴ org/ml): *Staphylococcus epidermidis, Staphylococcus aureus, Bacillus subtilis, Escherichia coli, Yersinia enterocolitica, Pseudomonas aeruginosa, Pseudomonas fluorescens*, and *Serratia marcescens*. The samples were divided into two sets. 0.1ml of "Double Dye" solution was added to each tube of one set, the other set was left untreated. The samples were incubated for 60min at 21°C, with constant mixing, under normal fluorescent light. The blood was filtered through a PVA filter column (Six 1.0cm thick pads), gravity flow, to remove the dyes. 10µl of each sample was spread on to trypticase soy agar. The plates were incubated overnight at 35°C. Colony counts were made on each plate and are shown in Table 3.

**Table 3**

| Colony counts | | | |
|---|---|---|---|
| | Control | Treated | Log red. |
| Staph epi | 102 | 0 | 4 |
| Staph aureus | 98 | 0 | 4 |
| Bacillus subtilis | 104 | 0 | 4 |
| E. coli | 250 | 5 | 4 |
| Y. enterocolitica | 92 | 0 | 4 |
| Pseudo aeruginosa | 101 | 2 | 4 |

### Virucidal Effect

Twelve 10.ml samples of whole blood were spiked with the following viruses: Vesicular Stomatitis Virus (VSV), Encephalomyocarditis Virus (EMCV), Bovine Viral Diarrhea Virus (BVDV), Porcine Parvovirus (PPV). Three tubes of each were prepared.

The samples were divided into three sets. 0.1ml of "Double Dye" solution was added to each tube of the first set, 0.1ml of "Double Dye" solution and 10mg of Sodium ascorbate was added to each tube of the second set and the third set was left untreated.

The samples were incubated for 60min at 21°C, with constant mixing under normal fluorescent light. The blood was filtered through a PVA filter column (Six 1.0cm thick pads), gravity flow.

Viral titers were determined using the Reed Muench variation of the viral endpoint assay and are shown in Table 4.

**Table 4**

| Viral titers | | | |
|---|---|---|---|
| | Control | Dye | Dye/Asc |
| VSV | 9 | 0 | 0 |
| EMCV | 6 | 0 | 0 |
| BVDV | 4.5 | 4.5 | 0 |
| PPV | 5.1 | 5.1 | 0 |

As can be seen from these results the double dye mixture was effective alone against some virus. The addition of ascorbate enhanced this effectiveness and resulted in a total kill of those virus not susceptible to double dye alone.

Double Dye treatment with ascorbate is an effective way to eliminate a large range of potentially infectious pathogens-including parasites, virus and bacteria-from the blood supply. The treatment is inexpensive and requires little specialized equipment or special skills.

## Claims

1. A method for disinfecting blood and blood fractions without using ultraviolet light treatment comprising the steps of :
contacting the blood or blood fraction with a mixture of crystal violet and methylene blue dyes and a reducing agent to form a blood-dye mixture;
mixing and incubating the blood-dye mixture; and
removing said dyes from the blood or blood fraction.

2. The method of Claim 1, wherein the methylene blue and crystal violet are present in essentially equal weight percentages.

3. The method of Claim 2, wherein the weight percentages are about 0,01%.

4. The method of Claim 3, wherein sufficient reducing agent is added to convert the mixture into a leuco form.

5. The method of claim 4, wherein the weight percentage of added reducing agent is at least equal to the combined weights of the added dyes.

6. The method of any of claims 1 to 5, wherein the reducing agent is ascorbate.

7. The method of Claim 1, wherein the step of removing includes passing the blood-dye mixture through a filter.

8. The method of Claims 1 or 7, wherein the filter comprises poly vinyl acetal and wherein said dyes bind to the polyvinyl acetal.

## Patentansprüche

1. Verfahren zum Desinfizieren von Blut und Blutfraktionen ohne Behandlung mit ultraviolettem Licht umfassend die Schritte:
Kontaktieren des Blutes oder der Blutfraktion mit einer Mischung von Kristallviolett- und Methylenblau-Farbstoffen und einem Reduktionsmittel, um eine Blut-Farbstoff-Mischung zu bilden;
Mischen und Inkubieren der Blut-Farbstoff-Mischung; und
Entfernen der Farbstoffe aus dem Blut oder der Blutfraktion.

2. Verfahren gemäß Anspruch 1, wobei Methylenblau und Kristallviolett in im Wesentlichen gleichen Gewichtsprozenten vorhanden sind.

3. Verfahren gemäß Anspruch 2, wobei die Gewichtsprozente etwa 0,01% sind.

4. Verfahren gemäß Anspruch 3, wobei ausreichend Reduktionsmittel hinzugefügt wird, um die Mischung in die Leuko-Form zu überführen.

5. Verfahren gemäß Anspruch 4, wobei das Gewichtsprozent des hinzugefügten Reduktionsmittels wenigstens den kombinierten Gewichten der hinzugefügten Farbstoffe entspricht.

6. Verfahren gemäß einem der Ansprüche 1 bis 5, wobei das Reduktionsmittel Ascorbat ist.

7. Verfahren gemäß Anspruch 1, wobei der Schritt des Entfernens das Passieren der Blut-Farbstoff-Mischung durch einen Filter beinhaltet.

8. Verfahren gemäß Anspruch 1 oder 7, wobei der Filter Polyvinylacetal beinhaltet und wobei die Farbstoffe an das Polyvinylacetal binden.

## Revendications

1. Procédé pour la désinfection de sang et de fractions sanguines sans l'utilisation d'un traitement à la lumière ultraviolette comprenant les étapes consistant :
de mise en contact du sang ou de la fraction sanguine avec un mélange de colorants de violet de méthyle et de bleu de méthylène et un agent réducteur pour former un mélange sang-colorant ;
de mélange et d'incubation du mélange sang-colorant ; et
d'élimination desdits colorants du sang ou de la fraction sanguine.

2. Procédé selon la revendication 1, dans lequel le bleu de méthylène et le violet de méthyle sont présents dans des pourcentages en poids essentiellement égaux.

3. Procédé selon la revendication 2, dans lequel les pourcentages en poids sont d'environ 0,01 %.

4. Procédé selon la revendication 3, dans lequel on ajoute suffisamment d'agent réducteur pour convertir le mélange dans une forme leuco.

5. Procédé selon la revendication 4, dans lequel le pourcentage en poids d'agent réducteur ajouté est au moins égal aux poids combinés des colorants ajoutés.

6. Procédé selon l'une quelconque des revendications 1 à 5, dans lequel l'agent réducteur est l'ascorbate.

7. Procédé selon la revendication 1, dans lequel l'étape d'élimination comprend le passage du mélange sang-colorant à travers un filtre.

8. Procédé selon les revendications 1 ou 7, dans lequel le filtre comprend du polyvinylacétal et dans lequel lesdits colorants se lient au polyvinylacétal.
